(19) Europäisches Patentamt / European Patent Office / Office européen des brevets

(11) **EP 2 943 114 B1**

(12) **EUROPÄISCHE PATENTSCHRIFT**

(45) Veröffentlichungstag und Bekanntmachung des Hinweises auf die Patenterteilung:
**01.08.2018 Patentblatt 2018/31**

(21) Anmeldenummer: **13826562.4**

(22) Anmeldetag: **23.12.2013**

(51) Int Cl.:
**G02B 3/06** (2006.01)     **A61B 3/00** (2006.01)
**A61B 3/103** (2006.01)

(86) Internationale Anmeldenummer:
**PCT/EP2013/003939**

(87) Internationale Veröffentlichungsnummer:
**WO 2014/108167 (17.07.2014 Gazette 2014/29)**

(54) **ABERROMETER (O.Ä.) MIT ASTIGMATISCHEM TARGET**

ABERROMETER (OR THE LIKE) HAVING AN ASTIGMATIC TARGET

ABERROMÈTRE (OU SIMILAIRE) À CIBLE ASTIGMATIQUE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **09.01.2013 DE 102013000295**

(43) Veröffentlichungstag der Anmeldung:
**18.11.2015 Patentblatt 2015/47**

(73) Patentinhaber: **Rodenstock GmbH**
**80687 München (DE)**

(72) Erfinder:
• **TRUMM, Stephan**
**80999 München (DE)**
• **ESSER, Gregor**
**80686 München (DE)**

(74) Vertreter: **Müller-Boré & Partner**
**Patentanwälte PartG mbB**
**Friedenheimer Brücke 21**
**80639 München (DE)**

(56) Entgegenhaltungen:
**WO-A2-2010/065475      US-A1- 2003 030 774**
**US-A1- 2004 032 568      US-A1- 2006 170 864**
**US-A1- 2012 287 398**

## Beschreibung

Die vorliegende Erfindung betrifft ein Verfahren und eine Vorrichtung zur Stimulation der Akkommodation zumindest eines Auges eines Probanden. Insbesondere bietet die vorliegende Erfindung damit eine besonders verlässliche Möglichkeit zur Bestimmung eines Satzes ophthalmologischer Daten zumindest eines Auges des Probanden und insbesondere zur Messung der Fehlsichtigkeit des Probanden und zur Ermittlung einer entsprechenden optischen Korrektur.

Zur Messung einer etwaigen Fehlsichtigkeit eines Augenpatienten oder Kunden (im Folgenden Probanden) und zur Ermittlung der erforderlichen, optischen Korrektur (Refraktionsbestimmung) beim Augenoptiker werden immer häufiger Autorefraktometer oder Aberrometer verwendet. Beispielsweise existieren im Stand der Technik Autorefraktometer, welche simulierte Sehzeichentafeln einsetzen und - analog einem Phoropter - dem Probanden zur subjektiven Refraktion und subjektiven Sehschärfebestimmung verschiedene Wirkungen vorsetzen. Für ein Autorefraktometer sei beispielsweise auf "Allergan Humphrey: Das Humphrey-Refraktometer, Produktinformationen (http://www.ophthalworld.de/cosmoshop/pix/a/media/21062012/Prospekt%20Autor ef%20580_%20585_%20590.pdf, Stand Juli 2012)" verwiesen.

Von der subjektiven Refraktion, die eine Auskunft des Probanden über die wahrgenommene Bildschärfe erfordert, ist die objektive Refraktion zu unterscheiden, die mittels einer apparativen Anordnung gemessen und durch die Brechungseigenschaften des Auges (insbesondere einschließlich der Hornhaut, der Linse und des Glaskörpers) bestimmt wird.

Wie in "K. Nicke und S. Trumm: Brillengläser der Zukunft - Schritt 3 Der DNEye Scanner, Der Augenoptiker, Juni 2012", beschrieben, werden dazu beispielsweise sogenannte virtuelle Targets auf Basis optischer Abbildungen in das Auge des Probanden projiziert, mit denen die Akkommodationszustände des Auges gesteuert werden können. Gemäß dem Stand der Technik wird diese Abbildung insbesondere durch eine oder mehrere sphärische Linsen realisiert. Ein herkömmliches, virtuelles Target stellt dabei insbesondere ein optisches Abbildungssystem dar, welches von virtuellen Objektpunkten auslaufende, sphärische Wellenfronten erzeugt, so dass diese auf das Auge des Probanden treffen. Damit erhält der Proband den (virtuellen) Eindruck eines realen Objekts in einer bestimmten Entfernung. Idealerweise ist die scheinbare Entfernung des dargestellten Objektes vom Auge des Probanden durch die sphärische Krümmung, also den Krümmungsradius der auf dem Auge auftreffenden Wellenfronten festgelegt. Durch eine Veränderung des Abbildungssystems des virtuellen Targets, insbesondere durch Veränderung des Krümmungsradius der auf dem Auge auftreffenden Wellenfronten, kann das Auge stimuliert werden, im Rahmen dessen physiologischer Möglichkeiten auf die unterschiedlichen Objektentfernungen zu akkommodieren. Dadurch kann das Auge bei unterschiedlichen, insbesondere gezielt einstellbaren Akkommodationszuständen untersucht werden und/oder es können die Grenzen der Akkommodationsfähigkeit des Auges ausgelotet werden.

Die Druckschrift US 2012/287398 A1 offenbart eine binokulare Vorrichtung zur Sehanalyse, um eine Verschreibung von Sehhilfen für die Augen eines Probanden zu bestimmen. Die Vorrichtung weist erste und zweite Targets auf, die jeweils dem ersten und dem zweiten Auge des Probanden zugeordnet sind. Mit Hilfe eines optischen Systems und zumindest einem Strahlteiler werden erste und zweite virtuelle Bilder der jeweiligen Targets zu den jeweiligen Augen des Probanden geführt. Des Weiteren weist die Vorrichtung eine erste und zweite sphärische Korrektureinrichtung sowie eine erste und zweite zylindrische Korrektureinrichtung auf, welche jeweils dem ersten und zweiten Auge zugeordnet sind.

Die Druckschrift WO 2010/065475 A2 offenbart ein Verfahren und eine Vorrichtung, um eine Refraktion eines Probanden zu bestimmen und somit eine Fehlsichtigkeit des Probanden zu korrigieren. Dazu werden zunächst eine zylindrische Refraktion mittels eines Aberrometers objektiv und anschließend ein Fokusfehler mittels eines Phoropters subjektiv ermittelt, wobei die subjektive Messung auf eine durch die objektive Messung zuvor ermittelte zylindrische Achse basiert.

Klassischerweise werden mit Geräten wie Autorefraktometern und Aberrometern augenoptische Messungen bzw. ophthalmologische Untersuchungen im fernakkommodierten Zustand durchgeführt, also bei entspannten Ziliarmuskeln. Dazu wird ein virtuelles Target mit Hilfe zumindest einer sphärischen Linse erzeugt, welches eine Abbildung eines Objekts in dem Auge des Probanden darstellt. Als Objekt werden dabei üblicherweise hinterleuchtete Diapositive eingesetzt. Die Stellung der zumindest einen Linse zum Dia und bei mehreren Linsen auch zueinander erlaubt es dabei, die Abbildung für den Grenzfall sogar so zu steuern, dass das Auge aufgrund der großen virtuellen Objektentfernung nicht mehr auf die Abbildung (also das virtuelle Objekt) akkommodieren kann, diese also in allen Richtungen nur als unscharf erkannt wird und sich der besagte entspannte Zustand des Auges einstellt. Dieses Vorgehen wird oft auch als "Nebelung" und der entsprechende entspannte Zustand als "genebelter Zustand" bezeichnet. Um eine solche Nebelung bei einem Probanden hervorzurufen ist es erforderlich, eine Abbildung zu erzeugen, die hinreichend jenseits des stärker positiven Hauptschnitts liegt. Dies wird, unabhängig von der Art der Fehlsichtigkeit, in einfacher Weise durch den Einsatz von Linsen mit geeigneter sphärischer Wirkung erzielt.

Vergleichsweise schwieriger und ungenauer gestalten sich augenoptische Messungen bzw. ophthalmologische Untersuchungen für die Nähe, d.h. im nahakkommodierten Zustand. Dies liegt zum einen daran, dass mit Hilfe eines virtuellen monokularen Targets zwar eine bestimmte optische Entfernung simuliert werden kann. Jedoch bleiben andere, den Akkommodationsmechanismus des Auges beeinflussende Faktoren, wie beispielsweise die binokulare Disparität (unterschiedliche Netzhautbilder der beiden Augen) und/oder zusätzliche Informationen aus der Szenerie (Ort und Größe des Objekts relativ zu anderen Objekten der Szenerie), unberücksichtigt. Zum anderen setzt eine erfolgreiche Nahmessung unter Verwendung eines virtuellen Targets voraus, dass der Proband während der Messung auch tatsächlich akkommodiert, d.h. dass er versucht, das Target scharf zu sehen.

Aus diesem Grund kann mit Hilfe eines virtuellen monokularen Targets, wie es in herkömmlichen Aberrometern und Autorefraktometern verwendet wird, oft nicht exakt die Akkommodationsleistung im Nahbereich (d.h. bei einem Blick in die Nähe) simuliert werden, die das Auge aufbringen würde, wenn es sich um ein reales Target in der entsprechenden Entfernung handeln würde. Eine nur auf einer vorgegebenen virtuellen Distanz (des Targets) basierende objektive Refraktionsmessung für den Akkommodationszustand, der beim Blick in diese Entfernung vorliegt, kann deshalb mit erheblichen Fehlern behaftet sein.

Es ist daher eine Aufgabe der vorliegenden Erfindung, eine Vorrichtung und ein Verfahren bereitzustellen, welche eine zuverlässige objektive Refraktionsmessung mittels eines Autorefraktometers, Aberrometers oder eines sonstigen augenoptischen bzw. ophthalmologischen Messgerätes, insbesondere für den Blick in die Nähe, ermöglichen.

Die Aufgabe wird gelöst durch eine Vorrichtung und ein Verfahren gemäß den unabhängigen Ansprüchen. Bevorzugte Ausführungsformen sind Gegenstand der Unteransprüche.

Somit bietet die vorliegende Erfindung in einem ersten Aspekt eine Vorrichtung zur Bestimmung eines Satzes ophthalmologischer Daten von zumindest einem Auge eines Probanden, insbesondere eine Vorrichtung zur objektiven Refraktionsbestimmung, aufweisend:

- eine Akkommodationsstimulanzeinrichtung, welche ausgelegt ist, ein virtuelles Target mit einer sphärischen Wirkung und mit einer einstellbaren zylindrischen Wirkung in das zumindest eine Auge des Probanden zu projizieren; und
- eine Messeinrichtung zum Erfassen ophthalmologischer Daten des zumindest einen Auges des Probanden, wobei

die Messeinrichtung ausgelegt ist, einen Wert einer astigmatischen Refraktion des zumindest einen Auges des Probanden zu erfassen und wobei die Vorrichtung eine Steuereinrichtung umfasst, welche ausgelegt ist, die sphärische Wirkung des virtuellen Targets von einer ersten sphärischen Wirkung zu einer zweiten sphärischen Wirkung zu verändern, während die Akkommodationsstimulanzeinrichtung das virtuelle Target mit einer zylindrischen Wirkung in das zumindest eine Auge des Probanden derart projiziert, dass die zylindrische Wirkung des virtuellen Targets den erfassten Wert der astigmatischen Fehlsichtigkeit des zumindest einen Auges zumindest teilweise kompensiert.

Im Kontext dieser Beschreibung werden unter "ophthalmologischen Daten" individuelle Parameter des zumindest einen Auges des Probanden verstanden. Insbesondere umfassen ophthalmologische Daten Sphäre und/oder Astigmatismus, besonders bevorzugt aber auch Abbildungsfehler höhere Ordnungen (z.B. Koma, sphärische Aberration, usw.). Zudem können ophthalmologische Daten alternativ oder zusätzlich auch pupillometrische Daten, d.h. Informationen zur Größe (beispielsweise in Form eines Radius), Gestalt und/oder zur Position (beispielsweise relativ zum Hornhautscheitel oder zur optischen Achse des Auges) einer Pupille und/oder Daten zur Beschreibung der Wellenfrontaberration des zumindest einen Auges umfassen.

Eine Akkommodationsstimulanzeinrichtung im Sinne dieser Erfindung ist ein optisches System, welches ausgelegt ist, ein virtuelles Objekt (Target) in das zumindest eine Auge des Probanden zu projizieren, um damit das Auge zum Akkommodieren auf eine durch das virtuelle Target festgelegte (virtuelle) Entfernung zu stimulieren. Mit anderen Worten bildet ein erfindungsgemäßes, virtuelles Target ein optisches Abbildungssystem, welches von virtuellen Objektpunkten auslaufende Wellenfronten erzeugt, so dass diese auf das Auge des Probanden treffen. Dabei weisen die von dem virtuellen Target erzeugten (jeweils einem virtuellen Objektpunkt entsprechenden) und auf das zumindest eine Auge des Probanden treffenden Wellenfronten neben einer (vorzugsweise einstellbaren) sphärischen Krümmung auch eine einstellbare zylindrische Krümmungskomponente auf. Diese zylindrische Krümmungskomponente ist vorzugsweise sowohl bezüglich des Betrags der Krümmung als auch bezüglich der Achselage einstellbar. Damit kann dann die zylindrische Krümmung der Wellenfronten derart eingestellt werden, dass sie eine individuelle astigmatische Refraktion des Auges des Probanden vollständig oder teilweise korrigiert. Bei vollständiger Akkommodation dieses Auges auf die virtuelle Objektentfernung (also die sphärische Krümmung der vom virtuellen Target erzeugten Wellenfronten) nimmt das Auge das virtuelle Objekt somit meist deutlich schärfer wahr, als dies ohne eine zumindest teilweise Kompensation der astigmatischen Refraktion der Fall wäre. Damit ist wiederum der Akkommodationszustand des Auges sehr viel präziser und besser reproduzierbar zu stimulieren.

Vorzugsweise kann die virtuelle Position des virtuellen Objekts (Targets) geändert werden, so dass auf diese Weise unterschiedliche Akkommodationszustände des zumindest einen Auges stimuliert werden können. Insbesondere kann vorzugsweise die Position des virtuellen Objekts zwischen einer Position zur Stimulation einer Fernakkommodation und einer Position zur Stimulation einer Nahakkommodation verändert werden. Zusätzlich kann die Position des virtuellen Objekts vorzugsweise derart eingestellt werden, dass das zumindest eine Auge des Probanden nicht mehr in der Lage ist, auf das virtuelle Objekt zu akkommodieren. In diesem Fall kann das virtuelle Objekt (Target) vom Probanden in allen Richtungen nur als unscharf wahrgenommen werden. Dies hat zur Folge, dass sich die Ziliarmuskeln entspannen. Ein derartiger Zustand wird als "genebelter" Zustand bezeichnet.

Als virtuelles Target wird insbesondere eine optische Projektion in bzw. auf das Auge des Probanden derart angesehen, dass diese Projektion auf der Netzhaut des Auges ein Abbild erzeugt, das dem Abbild eines realen Objekts in einer bestimmten Entfernung vom Auge entspricht. Diese bestimmte Entfernung wird für das virtuelle Target hier auch als virtuelle Position bezeichnet. Mit anderen Worten ist ein virtuelles Target im Sinne dieser Erfindung insbesondere eine Abbildung eines Objekts in das zumindest eine Auge des Probanden. Als Objekt kann beispielsweise ein hinterleuchtetes Diapositiv verwendet werden.

Da es sich bei dem virtuellen Target nicht (unmittelbar) um ein reales Objekt an der virtuellen Position handelt, kann durch geeignete Konstruktion des optischen Systems (der Akkommodationsstimulanzeinrichtung) zur Projektion auch eine virtuelle Position jenseits von unendlich simuliert werden. Dies entspricht dann Wellenfronten, die zum Auge hin (also in Propagationsrichtung) konvergieren.

Das virtuelle Target hat eine, vorzugsweise einstellbare, sphärische Wirkung. Abhängig vom Wert der sphärischen Wirkung des virtuellen Targets muss das zumindest eine Auge, in das das virtuelle Target projiziert ist, mehr oder weniger stark akkommodieren, damit der Proband das virtuelle Target scharf wahrnimmt.

Im Rahmen der vorliegenden Erfindung wurde erkannt, dass insbesondere für Probanden mit einer astigmatischen Komponente in der Fehlsichtigkeit häufig Fehler bei der Refraktionsbestimmung auftreten. Dies rührt insbesondere daher, dass für Probanden mit einer astigmatischen Komponente in der Fehlsichtigkeit ein virtuelles Target mit einer nur sphärischen Wirkung nicht vollständig und in jeder Richtung gleichmäßig scharf erscheint. Vielmehr kann es zum Springen zwischen zwei Akkommodationszuständen kommen. Mit anderen Worten kann in diesem Fall das Auge nicht zu einer eindeutigen Akkommodation stimuliert werden, was zu Fehlern bei der Ermittlung ophthalmologischer Daten, insbesondere bei der (objektiven) Refraktionsbestimmung, sowohl in der Fernakkommodation besonders aber in der Nahakkommodation führt.

Durch das erfindungsgemäße virtuelle Target, welches neben der sphärischen Wirkung auch eine - insbesondere nach Betrag und Richtung - einstellbare zylindrische bzw. astigmatische Wirkung aufweist, kann dieses Problem zumindest teilweise behoben und die Verlässlichkeit und Reproduzierbarkeit von erfassten ophthalmologischen Daten deutlich verbessert werden. Das virtuelle Target ermöglicht eine Kompensation des Astigmatismus des zumindest einen Auges des Probanden, so dass der Proband das virtuelle Target (abgesehen von den in diesem Zusammenhang vernachlässigbaren Aberrationen höherer Ordnung) in alle Richtungen scharf oder zumindest schärfer als bei herkömmlichen objektiven Refraktionsbestimmungen wahrnehmen kann. Dadurch wird für den Probanden nicht nur die Fixation, sondern insbesondere auch die Akkommodation erleichtert. Fehler bei der Ermittlung ophthalmologischer Daten, insbesondere bei der objektiven Refraktionsbestimmung, die dadurch entstehen, dass der Proband das virtuelle Target nicht richtig bzw. unzureichend fixiert und/oder nicht richtig bzw. unzureichend auf das Target akkommodiert, können auf diese Weise verringert oder vermieden werden. Auch bei der Fernrefraktion ist es vorteilhaft, wenn das zum Nebeln eingesetzte virtuelle Target auf der Netzhaut in allen Richtungen gleichmäßig unscharf ist und keine durch das Auge induzierte (und nicht kompensierte) astigmatische Komponente aufweist. Insbesondere ist der damit erzeugte genebelte Zustand für den Probanden angenehmer.

Die Messeinrichtung zum Erfassen ophthalmologischer Daten des zumindest einen Auges des Probanden umfasst vorzugsweise eine Beleuchtungseinrichtung, insbesondere einen Laser oder eine Laserdiode, zum Beleuchten eines Punktes auf der Netzhaut des Auges. Von diesem Punkt läuft dann eine Kugelwelle aus, welche an den optischen Grenzflächen des Auges (Glaskörper, Linsenflächen, Hornhaut) gebrochen wird. Vorzugsweise weist die Messeinrichtung auch einen Detektor zum Detektieren der Wellenfrontaberration zumindest eines Teils dieser resultierenden Wellen auf. Die ophthalmologischen Daten können aus dem detektierten Signal ermittelt werden.

Vorzugsweise weist die Akkommodationsstimulanzeinrichtung zumindest eine sphärische Linse oder ein sphärisches Linsensystem, das heißt eine Linse (bzw. ein Linsensystem) mit einer sphärischen Wirkung, und zumindest eine zylindrische Linse oder ein zylindrisches Linsensystem, das heißt eine Linse (bzw. ein Linsensystem) mit einer zylindrischen Wirkung, auf. Vorzugsweise sind die Linsen beweglich angeordnet. Die Stellung der zumindest einen sphärischen Linse zum abzubildenden Objekt (z.B. Diapositiv) und bei mehreren Linsen auch zueinander erlaubt es dabei, die virtuelle Position des virtuellen Targets festzulegen bzw. zu ändern.

Eine bevorzugte Ausführungsform der erfindungsgemäßen Vorrichtung zur Bestimmung eines Satzes ophthalmologischer Daten betrifft die vorstehend beschriebene Vorrichtung, wobei die Akkommodationsstimulanzeinrichtung ein Magazin, beispielsweise ein Revolvermagazin, mit einer Vielzahl von Zylinderlinsen umfasst, welche jeweils unterschiedliche zylindrische Wirkungen aufweisen. Vorzugsweise ist das Magazin derart ausgelegt und angeordnet, dass einzelne Zylinderlinsen oder eine Kombination mehrerer Zylinderlinsen des Magazins auswählbar und zum Projizieren des virtuellen Targets verwendbar sind.

Vorzugsweise werden die Zylinderlinsen automatisch ausgewählt und zusätzlich zu den Linsen, die für die sphärische Wirkung des virtuellen Targets verantwortlich sind, in den Strahlengang zum Abbilden des Objekts in das zumindest eine Auge des Probanden eingesetzt. Mit anderen Worten können die verschiedenen Zylinderlinsen des Magazins dem zumindest einen Auge des Probanden wahlweise vorgehalten werden. Die Auswahl der Zylinderstärke erfolgt dabei über die Auswahl der Zylinderlinsen. Das Magazin kann beispielsweise Linsen mit einer Abstufung von 0,25 dpt oder 0,125 dpt enthalten. Die Zylinderlinsen decken vorzugsweise einen Bereich der zylindrischen Wirkung ab, dessen Betrag sich zumindest von etwa 0 bis etwa 2 dpt, vorzugsweise zumindest von etwa 0 bis etwa 4 dpt, noch mehr bevorzugt zumindest von etwa 0 bis etwa 6 dpt insbesondere zumindest teilweise in der oben genannten Abstufung abdeckt. Besonders bevorzugt sind auch Linsen mit einer zylindrischen Wirkung mit einem Betrag im Bereich von etwa 8 dpt bis etwa 10 dpt oder mehr vorgesehen. Dabei ist es ausreichend nur positive oder nur negative Wirkungen vorzuhalten, da bei geeigneter Wahl der sphärischen Wirkung $s_G$ des Grundsystems (das Grundsystem umfasst keine Linsen des Magazins) jedes übliche bzw. erforderliche sphärische Äquivalent $s$ realisierbar ist. Die Zylinderstärke $c$ entspricht dann der Zylinderstärke der aus dem Magazin $c_M$ gewählten Zylinderlinse. Das sphärische Äquivalent ergibt sich somit zu

$$s = s_G + c_M \qquad\qquad c = c_M \,.$$

Analog können unterschiedliche Achslagen in Kombination mit den Wirkungen als jeweils einzelne Linse vorgehalten werden. Vorzugsweise kann die ausgewählte Linse vor dem Einsetzen in den Strahlengang oder innerhalb des Strahlengangs durch Rotation entlang der optischen Achse entsprechend ausgerichtet werden.

Vorzugsweise umfasst die Akkommodationsstimulanzeinrichtung ein Alvarez-Linsensystem. Eine Alvarez-Linse ist beispielsweise in H. Paul: Lexikon der Optik, Spektrum Akademischer Verlag GmbH (2003) (online unter: www.wissenschaft-online.de/abo/lexikon/optik/130, Stand Mai 2012) beschrieben. Ein Alvarez-Linsensystem (Alvarez-Linse) besteht aus zwei Linsenelementen, die entlang einer gemeinsamen optischen Achse (z-Richtung) angeordnet sind und relativ zueinander verschiebbar sind. Die gekrümmten Flächen der beiden Linsenelemente können durch die Gleichung

$$z = axy^2 + (a/3)x^3 + bx \qquad (2)$$

beschrieben werden. Hierbei bezeichnen $x$, $y$ und $z$ kartesische Koordinaten des Linsensystems, wobei die z-Richtung eine gemeinsame Achse der beiden Linsenelemente und damit die optische Achse definiert. Weiterhin sind $a$ und $b$ Konstanten. Durch eine relative Translationsbewegung der beiden Linsenelemente senkrecht zur optischen Achse (d.h. in die x- bzw. y-Richtung) kann sowohl die sphärische (bei einer relativen Bewegung der Linsenelemente entlang der x-Richtung) als auch die zylindrische (bei einer relativen Bewegung der Linsenelemente entlang der zur x- und z-Richtung senkrechten y-Richtung) Wirkung des Alvarez-Linsensystems verändert werden. Mit anderen Worten ermöglicht ein Alvarez-Linsensystem eine kontinuierliche Veränderung der sphärozylindrischen Wirkung. Die beiden Linsenelemente neutralisieren sich bei genauer Überdeckung zur Nullwirkung. Bei gegenseitiger Verschiebung der Linsenelemente in x-Richtung entstehen nach der einen Seite positive, nach der anderen Seite negative optische Wirkungen (sphärisch). Bei gegenseitiger Verschiebung in y-Richtung resultieren variable Zylinderwirkungen, und bei schrägen Verschiebungen ergeben sich in Abhängigkeit von Größe und Richtung der Verschiebung sphärozylindrische Kombinationen.

Eine Alvarez-Linse hat somit den Vorteil, dass Sie nicht nur hinsichtlich der zylindrischen, sondern auch der sphärischen Wirkung variabel einstellbar ist. Wird auf die Veränderung der sphärischen Wirkung kein Wert gelegt, sondern diese durch zusätzliche sphärische Linsen und/oder eine Bewegung des Alvarez-Linsensystems entlang der optischen Achse (z-Richtung) in Relation zu den anderen optischen Elementen des Systems realisiert, kann eine Translation entlang der Richtung der sphärischen Wirkung (x-Achse) entfallen. Zum Einstellen der Richtung der Zylinderachse kann die Alvarez-Linse drehbar um die optische Achse (z-Achse) gelagert sein.

In einer bevorzugten Ausführungsform umfasst die Akkommodationsstimulanzeinrichtung zumindest zwei gegeneinander verdrehbare Linsen mit jeweils zumindest einer zylindrischen Komponente in den Wirkungen. Vorzugsweise sind diese Linsen koaxial zueinander angeordnet und um die um die gemeinsame optische Achse gegeneinander verdrehbar. Vorzugsweise sind sie jeweils unabhängig voneinander um die gemeinsame koaxiale Achse drehbar gelagert und sie haben besonders bevorzugt betragsmäßig gleiche zylindrische Wirkungen.

Durch die Rotation der beiden koaxial angeordneten Linsen gegeneinander lässt sich die Stärke des Zylinders (zylindrische Wirkung) einstellen. Durch eine gemeinsame Rotation der beiden Linsen lässt sich die Achslage einstellen. Nach "Breitenstein: Allgemeine Optik, Skript zur Vorlesung an der HFA Köln (2004) (online: www.hfak.de/dozenten/breitenstein/Skripte/AlOp1.pdf)" ergibt sich die kombinierte Wirkung $(c_K, a_K)$ zweier Linsen mit astigmatischer Wirkung und den zylindrischen Komponenten $c_1$ und $c_2$ in Abhängigkeit von den Achslagen $a_1$ und $a_2$ in der Näherung dünner Linsen und bei vernachlässigbarem Abstand zu

$$c_K = \sqrt{c_1^2 + c_2^2 + 2c_1 c_2 \cos\big(2(a_1 - a_2)\big)} \qquad (3)$$

In dieser Darstellung wird vereinfacht die Pluszylinderschreibweise verwendet. Es gilt also: $c_1, c_2 \geq 0$.

Bevorzugt besitzen beide Linsen denselben Betrag der zylindrischen Wirkung, da nur so eine ideale Auslöschung der zylindrischen Komponenten möglich ist. Weiterhin bevorzugt ist der Betrag der zylindrischen Wirkung für beide Linsen jeweils die Hälfte des Betrages der maximal zu erreichenden zylindrischen Wirkung $\left( c_1 = c_2 = \dfrac{c_{Max}}{2} \right)$ oder geringfügig größer. So kann der vollständige Bereich in der zylindrischen Wirkung von Null bis zu einem gewünschten Maximalwert realisiert werden. Die obige Gleichung (3) vereinfacht sich somit zu

$$c_K = \frac{1}{2} c_{Max} \cdot \sqrt{2 + 2\cos\big(2(a_1 - a_2)\big)} \qquad (4)$$

Vorzugsweise sind die Achslagen $a_1$ und $a_2$ der beiden koaxial angeordneten Linsen gegeben durch

$$a_{1,2} = a_K \pm \frac{1}{4} \cdot \arccos\left( \frac{2c_K^2 - c_{Max}^2}{c_{Max}^2} \right) \qquad (5)$$

mit der kombinierten Achslage $a_K$, der kombinierten zylindrischen Wirkung $c_K$ und des maximal zu erreichenden Betrages der zylindrischen Wirkung $c_{Max}$.

Vorzugsweise sind beide koaxial angeordnete Linsen positive zylindrische Linsen gleicher Stärke (mit gleicher zylindrischer Wirkung) oder negative zylindrische Linsen gleicher Stärke oder eine positive und eine negative zylindrische Linse. Unter einer zylindrischen Linse wird eine Linse verstanden, die im betragsmäßig geringeren Hauptschnitt keine Wirkung aufweist. Ihre Stärke ist analog als Wirkung im betragsmäßig größeren Hauptschnitt definiert.

In einer bevorzugten Ausführungsform weisen die zwei Linsen ineinander fassende rotationssymmetrische Flächen, vorzugsweise Planflächen, auf, wobei diese Flächen einander zugewandt sind. Diese Ausführungsform hat den Vorteil, dass sich dadurch ein geringer Abstand zwischen den Linsen realisieren lässt.

In einer bevorzugten Ausführungsform weist die Akkommodationsstimulanzeinrichtung eine positive und eine negative Zylinderlinse mit entgegengesetzt gleicher Wirkung auf, welche gegeneinander drehbar gelagert und vorzugsweise gegeneinander verschiebbar sind.

Vorzugsweise weist die Akkommodationsstimulanzeinrichtung eine sogenannte Stokessche Linse auf, mit welcher stufenlos bestimmte astigmatische Wirkungen eingestellt werden können. Eine Stokessche Linse ist eine Kombination aus einer positiven und einer negativen Zylinderlinse mit entgegengesetzt gleicher Stärke bzw. Wirkung. Die hierbei zum Einsatz kommenden Zylinderlinsen weisen vorzugsweise jeweils eine Planfläche und eine positiv bzw. negativ gekrümmten Zylinderfläche auf.

Eine Stokessche Linse ist beispielsweise in "H. Paul: Lexikon der Optik, Spektrum Akademischer Verlag GmbH (2003) (online: www.wissenschaft-online.de/abo/lexikon/optik/220)" beschrieben. Gleichung (4) für die Stärke $c_K$ des resultierenden Zylinders vereinfacht sich im Falle einer Stokesschen Linse zu

$$c_K = c_{Max} \cdot \sin(\Delta a) \qquad (6)$$

wobei $\Delta a$ der Winkel zwischen der Zylinderachse $a_p$ der positiven Zylinderlinse und der Zylinderachse $a_n$ der negativen Zylinderlinse ist. Unter der Zylinderachse wird bei Zylinderlinsen in diesem Zusammenhang insbesondere die Achse mit der Wirkung (unabhängig vom Vorzeichen der Wirkung) verstanden.

Vorzugsweise ist zumindest eine Linse der oben beschriebenen Linsensysteme entlang der optischen Achse verschiebbar. Besonders bevorzugt sind sämtliche Linsen der oben beschriebenen Linsensysteme entlang der optischen Achse verschiebbar. Eine etwaige Änderung der Stärke der sphärischen Wirkung kann dann bei der Berechnung der zum Erreichen von vorgegeben sphärischen Wirkungen des virtuellen Targets notwendigen Positionen relativ zu anderen optischen Komponenten der Akkommodationsstimulanzeinrichtung berücksichtigt werden.

Vorzugsweise ist die Vorrichtung zur Bestimmung eines Satzes ophthalmologischer Daten als Aberrometer und/oder Autorefraktometer ausgestaltet. Weiter bevorzugt umfasst die Vorrichtung zusätzlich einen Wellenfrontsensor, beispielsweise einen Shack-Hartmann Sensor, zur Bestimmung der Wellenfront des zumindest einen Auges des Probanden.

Vorzugsweise ist die Messeinrichtung ausgelegt, eine astigmatische Refraktion (also eine Fehlsichtigkeit) des zumindest einen Auges des Probanden zu erfassen, insbesondere als zumindest ein Teil von ophthalmologischen Daten zu messen. Darüber hinaus umfasst die Vorrichtung vorzugsweise eine Steuereinrichtung, welche ausgelegt ist, die zylindrische Wirkung des virtuellen Targets vorzugsweise automatisch derart einzustellen, dass damit die erfasste astigmatische Refraktion des zumindest einen Auges zumindest teilweise, vorzugsweise sogar zumindest größtenteils oder im Wesentlichen vollständig kompensiert wird. Damit kann eine Bestimmung von ophthalmologischen Daten für einen Probanden sehr zuverlässig zumindest weitgehend automatisiert und objektiviert vorgenommen werden, indem die Einstellung der gewünschten bzw. erforderlichen zylindrischen Wirkung des virtuellen Targets vorzugsweise automatisch erfolgt. In anderen Ausführungsformen, kann die zylindrische Wirkung des virtuellen Targets auch von einem Benutzer beispielweise auf Basis eines bereits früher ermittelten Wertes der astigmatischen Refraktion des Probanden (insbesondere manuell) eingestellt werden.

Besonders bevorzugt ist die Vorrichtung ausgelegt, nacheinander verschiedene virtuelle Entfernungen des virtuellen Targets einzustellen und für jede virtuelle Entfernung ophthalmologische Daten des zumindest einen Auges zu erfassen. Dabei wird besonders bevorzugt mit jedem erfassten Datensatz, also bei jeder virtuellen eingestellten Entfernung (außer eventuell beim letzten Messschritt), eine astigmatische Refraktion des zumindest einen Auges erfasst und die zylindrische Wirkung des virtuellen Target bei der jeweils nachfolgenden virtuellen Entfernung gemäß der erfassen astigmatischen Refraktion eingestellt. In anderen Wort wird somit die in einem ersten Schritt (also bei einer ersten virtuellen Entfernung) erfasste astigmatische Refraktion herangezogen, um darauf basierend die zylindrische Wirkung des virtuellen Targets für einen (unmittelbar nachfolgenden) zweiten Schritt (also bei einer zweiten virtuellen Entfernung) derart einzustellen, dass damit die erfasste astigmatische Refraktion in der beschriebenen Weise zumindest teilweise kompensiert wird. Dies ist besonders dann vorteilhaft, wenn der Unterschied nacheinander eingestellter virtueller Entfernungen nicht zu groß ist. Besonders bevorzugt beginnt die Vorrichtung (bzw. ein entsprechendes Verfahren zur Stimulation der Akkommodation) bei einer großen virtuellen Entfernung, insbesondere in einem "genebelten" Zustand und verkleinert die virtuelle Entfernung von Messung zu Messung sukzessive (vorzugsweise kontiniuerlich), vorzugsweise bis hin zu einer Nahakkommodationsgrenze, bei der die Akkommodation des Auge der weiteren Verkleinerung der virtuellen Entfernung nicht mehr folgen kann.

In einem weiteren Aspekt bietet die Erfindung ein Verfahren zur Stimulation der Akkommodation von zumindest einem Auge eines Probanden. Das Verfahren umfasst ein Erfassen eines (ersten) Wertes einer astigmatischen Fehlsichtigkeit (astigmatische Refraktion) des zumindest einen Auges des Probanden. Soweit noch nicht feststeht, ob und welche astigmatische Fehlsichtigkeit das zu untersuchende Auge des Proband hat, wird diese vorzugsweise zunächst gemessen oder abgeschätzt. Besonders bevorzugt wird der (erste) Wert der astigmatischen Fehlsichtigkeit insbesondere mittels einer erfindungsgemäßen Vorrichtung (beispielsweise bei einer Voreinstellung des virtuellen Targets mit einer zylindrischen Wirkung von Null) bei einer Fernakkommodation des Auges und/oder im genebelten Zustand gemessen. Soweit bereits früher eine Messung der astigmatischen Fehlsichtigkeit durchgeführt wurde und diese eventuell sogar schon für eine vorhandene Brille benutzt wurde, kann auf diese bekannten Werte zurückgegriffen werden.

Erfindungsgemäß wird nun ein virtuelles Target mit einer (ersten) sphärischen Wirkung und mit einer einstellbaren (ersten) zylindrischen Wirkung derart bereitgestellt und in das zumindest eine Auge des Probanden projiziert, dass die (erste) zylindrische Wirkung des virtuellen Targets den erfassten (ersten) Wert der astigmatischen Fehlsichtigkeit des zumindest einen Auges zumindest teilweise kompensiert.

In einer bevorzugten Ausführungsform umfasst das Verfahren eine Vormessung, welche bereits einen Schritt des Projizierens eines virtuellen Targets mit sphärischer (und einstellbarer zylindrischer) Wirkung in das zumindest eine Auge des Probanden umfasst, während das Auge auf das virtuelle Target akkommodiert ist oder ein genebelter Zustand des Auges eingenommen ist (das Auge also nicht vollständig akkommodieren kann). Dazu umfasst das Verfahren vorzugsweise einen Schritt des Erfassens bzw. Ermittelns des ersten Wertes der astigmatischen Fehlsichtigkeit des zumindest einen Auges des Probanden. Vorzugsweise wird dabei neben der astigmatischen Fehlsichtigkeit auch ein (erster) Wert der sphärischen Fehlsichtigkeit des zumindest einen Auges des Probanden erfasst. Vorzugsweise wird das Erfassen eines ersten Wertes einer astigmatischen Fehlsichtigkeit und/oder einer sphärischen Fehlsichtigkeit des zumindest einen Auges des Probanden im genebelten Zustand durchgeführt. Der genebelte, d.h. entspannte Zustand kann dadurch realisiert werden, dass die virtuelle Entfernung des virtuellen Targets vom zumindest einen Auge des Probanden hinreichend groß gewählt wird, so dass das zumindest eine Auge nicht mehr in der Lage ist, auf das virtuelle Target zu akkommodieren und der Proband das virtuelle Target nur unscharf wahrnehmen kann. Besonders bevorzugt wird die Vormessung im nur leicht genebelten Zustand durchgeführt, d.h. bei einer virtuellen Position des virtuellen Targets, bei der das zumindest eine Auge des Probanden gerade nicht mehr in der Lage ist, auf das virtuelle Target zu akkommodieren.

Nach dem Schritt des Erfassens eines ersten Wertes einer astigmatischen Fehlsichtigkeit des zumindest einen Auges des Probanden insbesondere in einer Vormessung umfasst das Verfahren einen Schritt des Einstellens der zylindrischen Wirkung des virtuellen Targets auf einen Wert, der den erfassten ersten Wert der astigmatischen Fehlsichtigkeit des zumindest einen Auges des Probanden im Wesentlichen kompensiert. Das Einstellen der astigmatischen Wirkung des virtuellen Targets kann beispielsweise durch eine Auswahl von entsprechenden Linsen aus einem Magazin der Akkommodationsstimulanzeinrichtung erfolgen. Alternativ kann das Einstellen der astigmatischen Wirkung des virtuellen Targets durch ein Einstellen der relativen Position bzw. ein Ausrichtung von Linsen eines Linsensystems (z.B. Alvarez-Linse oder Stokessche Linse) erfolgen.

In einem bevorzugten Aspekt bietet die Erfindung ein Verfahren zur Bestimmung eines Satzes ophthalmologischer Daten von zumindest einem Auge eines Probanden. Dabei wird zunächst die Akkommodation des zumindest einen Auges in der erfindungsgemäßen Weise mittels eines virtuellen Targets mit einstellbarer zylindrischer Wirkung stimuliert. Während das Auge auf das entsprechend eingestellte virtuelle Target akkommodiert, werden die ophthalmologischen Daten des Auges erfasst.

Nach dem Schritt des Einstellens der zylindrischen (astigmatischen) Wirkung des virtuellen Targets auf einen Wert, der den erfassten ersten Wert der astigmatischen Fehlsichtigkeit des zumindest einen Auges des Probanden im Wesentlichen kompensiert umfasst das Verfahren somit vorzugsweise einen Schritt des Erfassens ophthalmologischer Daten des zumindest einen Auges des Probanden in einer Hauptmessung (z.B. zweiten Messung). Im Vergleich zu einer ersten Messung (Vormessung) dient die zweite Messung (Hauptmessung) insbesondere zum genaueren Bestimmen ophthalmologischer Daten, insbesondere der Refraktion des zumindest einen Auges des Probanden und wird daher hier auch als Hauptmessung bezeichnet. Um die Genauigkeit der gemessenen Werte zu erhöhen bzw. um etwaige Messfehler zu reduzieren werden vorzugsweise eine Vielzahl von Hauptmessungen durchgeführt und deren Ergebnisse statistisch gemittelt.

Vorzugsweise erfolgt das Erfassen der ophthalmologischen Daten des zumindest einen Auges des Probanden bei einer Nahakkommodation des zumindest einen Auges des Probanden. Insbesondere wird dazu zum Erfassen der ophthalmologischen Daten des zumindest einen Auges des Probanden das virtuelle Target in eine bestimmte bzw. vorgegebene virtuelle Entfernung zu dem zumindest einen Auge des Probanden gebracht. Im Allgemeinen kann das virtuelle Target in eine virtuelle Position zur Fernakkommodation oder Nahakkommodation gebracht werden. Das virtuelle Target kann aber auch in eine virtuelle Position gebracht werden, die einen genebelten Zustand bewirkt.

In einer bevorzugten Ausführungsform umfasst das Erfassen der ophthalmologischen Daten ein Erfassen eines zweiten Wertes der astigmatischen Fehlsichtigkeit des zumindest einen Auges. Besonders bevorzugt umfasst das Verfahren dabei ferner ein Projizieren des virtuellen Targets mit einer zweiten sphärischen Wirkung, welche auch mit der ersten sphärischen Wirkung übereinstimmen kann, sich aber vorzugsweise davon unterscheidet, und einer zweiten zylindrischen Wirkung in das zumindest eine Auge derart, dass die zweite zylindrische Wirkung des virtuellen Targets den erfassten zweiten Wert der astigmatischen Fehlsichtigkeit zumindest teilweise kompensiert. Weiter bevorzugt umfasst das Verfahren in diesem Fall ein Erfassen weiterer ophthalmologischer Daten des zumindest einen Auges, während das Auge zur Akkommodation auf das virtuelle Target mit der zweiten zylindrischen Wirkung stimuliert wird. Dieser Vorgang kann auch mehrmals wiederholt werden, indem die weiteren ophthalmologischen Daten wiederum eine astigmatische Fehlsichtigkeit des Auges umfassen, auf welche die zylindrische Wirkung des Targets für einen wiederum anschließenden Schritte des Erfassens ophthalmologischer Daten bei einem weiteren Akkommodationszustand des Auges (aufgrund einer weiteren virtuellen Entfernung des virtuellen Targets) eingestellt wird.

Insbesondere umfasst das Verfahren somit vorzugsweise einen Schritt des Erfassens ophthalmologischer Daten des zumindest einen Auges des Probanden in zumindest einer weiteren (d.h. einer dritten, vierten, fünften, usw.) Messung, welche bei einer zu den vorangegangenen Messungen unterschiedlichen optischen (virtuellen) Entfernung des virtuellen Targets zu dem zumindest einen Auge des Probanden durchgeführt wird, wobei vor Durchführung der zumindest einen weiteren (d.h. der dritten, vierten, fünften, usw.) Messung die einstellbare zylindrische Wirkung des virtuellen Targets vorzugsweise auf einen Wert eingestellt ist bzw. wird, der den bei der jeweils vorangegangenen (zweiten, dritten, vierten, usw.) Messung erfassten Wert der astigmatischen Fehlsichtigkeit des zumindest einen Auges des Probanden im Wesentlichen kompensiert.

Wie die zweite Messung dient auch die zumindest eine weitere (dritte, vierte, fünfte, usw.) Messung zur genauen Bestimmung ophthalmologischer Daten, insbesondere der Refraktionsbestimmung, so dass auch die zumindest eine weitere (dritte, vierte, fünfte, usw.) Messung als Hauptmessung bezeichnet werden könnte. Mit anderen Worten wird die zylindrische Wirkung des virtuellen Targets vor jeder Hauptmessung vorzugsweise auf Basis einer (insbesondere unmittelbar) vorangegangenen Messung derart angepasst, dass die durch die vorangegangene Messung ermittelte astigmatische Fehlsichtigkeit des zumindest einen Auges des Probanden im Wesentlichen kompensiert wird. Vorzugsweise wird die zweite Messung bei einer virtuellen Position des virtuellen Targets durchgeführt, welche einen genebelten Zustand bewirkt. Für jede weitere (dritte, vierte, fünfte, usw.) Messung kann dann die optische Entfernung des virtuellen Targets sukzessive verringert werden.

In einer bevorzugten Ausführungsform erfolgt das Erfassen ophthalmologischer Daten des zumindest einen Auges des Probanden in der zweiten Messung kontinuierlich während eines monotonen Verschiebens des virtuellen Targets von einer Position zur Stimulation einer Fernakkommodation über eine Position zur Stimulation einer Nahakkommodation bis hin zu einer Position zur Erzeugung eines genebelten Zustandes. Vorzugsweise beginnt die zweite Messung bei einer hinreichend großen virtuellen Entfernung des virtuellen Targets, welche einen genebelten Zustand bewirkt.

Vorzugsweise werden während des gesamten Vorgangs sämtliche gemessenen Werte zu jeder virtuellen Position des Targets protokolliert. Am Ende des Messvorgangs kann dann die Messung ausgewählt werden, bei der die maximale Akkommodationsleistung des zumindest einen Auges des Probanden erreicht wurde. Die gewonnenen Daten dieser Messung können als Ergebnis der Nahmessung verwendet werden. Auf diese Weise kann eine vollständige Messung aus Wellenfront, Pupillengröße sowie den Abbildungsfehlern höherer und niederer Ordnung für den Blick in die Nähe realisiert werden.

Das erfindungsgemäße Verfahren umfasst ein (vorzugsweise im Wesentlichen kontinuierliches) Verändern der sphärischen Wirkung des virtuellen Targets von der ersten sphärischen Wirkung zu einer zweiten sphärischen Wirkung, während das virtuelle Target mit einer zylindrischen Wirkung in das zumindest eine Auge des Probanden derart projiziert wird, dass die zylindrische Wirkung des virtuellen Targets den erfassten ersten Wert der astigmatischen Fehlsichtigkeit des zumindest einen Auges zumindest teilweise kompensiert.

Es ist dabei nicht notwendigerweise erforderlich, dass an jedem Wert oder auch nur an einer Vielzahl von Werten der sphärischen Wirkung eine Messung von ophthalmologischen Daten erfolgt. Es ist auch nicht unbedingt erforderlich, dass während der Veränderung der sphärischen Wirkung die zylindrische Wirkung nachgeregelt wird.

In einer bevorzugten Ausführungsform kann zum Beispiel ein Verändern der sphärischen Wirkung von einem Zustand der Fernakkommodation oder einem genebelten Zustand hin zu einem Zustand der Nahakkommodation erfolgen, wobei im Zustand der Nahakkommodation dann eine objektive Messung von ophthalmologischen Daten erfolgt. Dabei wird in einer bevorzugten Ausführungsform die zylindrische Wirkung des virtuellen Targets beibehalten. Durch die zumindest teilweise Kompensation der astigmatischen Fehlsichtigkeit während der Projektion des virtuellen Targets kann das Auge in sehr präzise reproduzierbarer Weise zur Akkommodation im Übergang von einem entspannten Zustand zu einem nahakkommodierten Zustand stimuliert werden. Damit wird die Messung der ophthalmologischen Daten in der Nahakkommodation wesentlich verbessert.

In einer weiteren bevorzugten Ausführungsform wird während der (kontinuierlichen) Veränderung der sphärischen Wirkung auch eine (kontinuierliche) Veränderung der zylindrischen Wirkung des virtuellen Targets bewirkt. Dabei kann die Veränderung der zylindrischen Wirkung beispielsweise in der bereits oben beschriebenen Weise auf einer (quasi-kontinuierlichen) Messung der astigmatischen Fehlsichtigkeit oder auf einer festen Modellvorgabe beruhen.

Bevorzugte Ausführungsformen der Erfindung werden nachfolgend unter Bezugnahme auf die beigefügten Zeichnungen beispielhaft erläutert. Dabei weisen die einzelnen beschriebenen Ausführungsformen zum Teil Merkmale auf, die nicht zwingend erforderlich sind, um den beanspruchten Gegenstand auszuführen, die aber in bestimmten Anwendungsfällen gewünschte Eigenschaften bereit stellen. So sollen auch Ausführungsformen als unter die beschriebene technische Lehre fallend offenbart angesehen werden, die nicht alle Merkmale der im Folgenden beschriebenen Ausführungsformen aufweisen. Ferner werden, um unnötige Wiederholungen zu vermeiden, bestimmte Merkmale nur in Bezug auf einzelne der im Folgenden beschriebenen Ausführungsformen erwähnt. Es wird darauf hingewiesen, dass die einzelnen Ausführungsformen daher nicht nur für sich genommen sondern auch in einer Zusammenschau betrachtet werden sollen. Anhand dieser Zusammenschau wird der Fachmann erkennen, dass einzelne Ausführungsformen auch durch Einbeziehung von einzelnen oder mehreren Merkmalen anderer Ausführungsformen modifiziert werden können. Es wird darauf hingewiesen, dass eine systematische Kombination der einzelnen Ausführungsformen mit einzelnen oder mehreren Merkmalen, die in Bezug auf andere Ausführungsformen beschrieben werden, wünschenswert und sinnvoll sein kann, und daher in Erwägung gezogen und auch als von der Beschreibung umfasst angesehen werden soll. Insbesondere ist die erfindungsgemäße Vorrichtung vorzugsweise ausgelegt, eines der in dieser Beschreibung dargelegten Verfahren auszuführen. Im Hinblick auf die nachfolgende Beschreibung bevorzugter Ausführungsformen zeigt:

Fig. 1     eine schematische Darstellung einer Vorrichtung gemäß einer bevorzugten Ausführungsform der vorliegenden Erfindung;

Fig. 2     eine schematische Darstellung eines Linsensystems (Stokessche Linse), welches gemäß einer bevorzugten Ausführungsform der vorliegenden Erfindung zur Astigmatismuskompensation in der Akkommodationsstimulanzeinrichtung eingesetzt werden kann; und

Fig. 3     eine schematische Darstellung eines Ablaufs eines Verfahrens gemäß einer bevorzugten Ausführungsform der vorliegenden Erfindung.

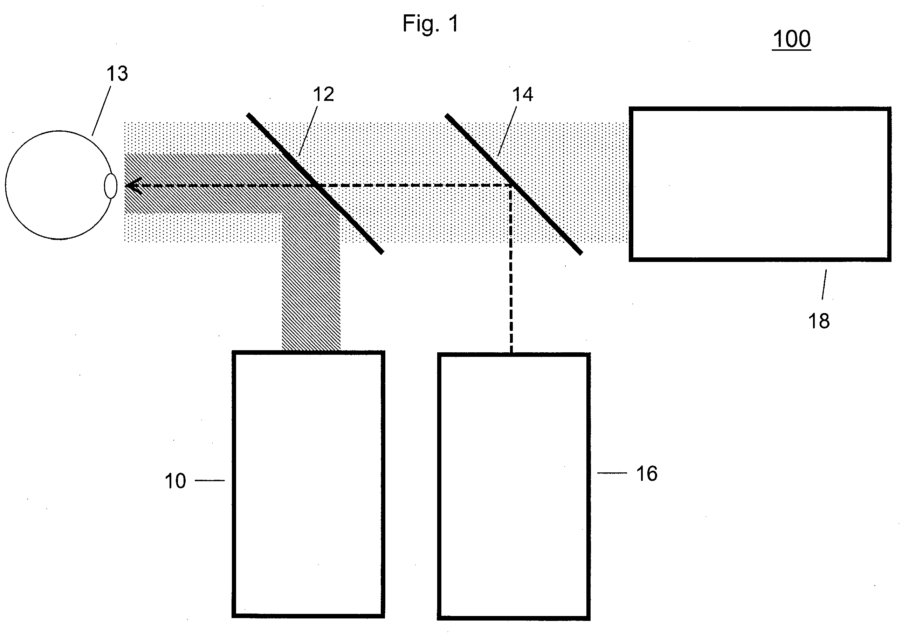

**Fig. 1** zeigt eine schematische Darstellung einer Vorrichtung zur Bestimmung ophthalmologischer Daten von zumindest einem Auge eines Probanden gemäß einer bevorzugten Ausführungsform der vorliegenden Erfindung. Die Vorrichtung umfasst eine Akkommodationsstimulanzeinrichtung 10, mit der ein virtuelles Target erzeugt und über einen ersten Strahlteiler 12 in das zumindest eine Auge 13 des Probanden projiziert wird. Das virtuelle Target ist dementsprechend eine Abbildung eines Objekts, vorzugsweise eines hinterleuchteten Diapositivs, auf die Netzhaut des zumindest einen Auges 13 des Probanden.

Mit Hilfe des virtuellen Targets kann eine Akkommodation des zumindest einen Auges des Probanden stimuliert werden. Die Stärke der stimulierten Akkommodation hängt von der virtuellen Position des virtuellen Targets bzw. von der virtuellen Entfernung des virtuellen Targets von dem zumindest einen Auge 13 des Probanden ab, welche mit der Akkommodationsstimulanzeinrichtung 10 eingestellt werden kann.

Die Akkommodationsstimulanzeinrichtung 10 umfasst vorzugsweise im Wesentlichen das Diapositiv (Dia), eine Beleuchtungseinrichtung zum Hinterleuchten des Diapositivs, sowie ein optisches System mit zumindest einer sphärischen Linse. Das optische System kann das hinterleuchtete Diapositiv auf die Netzhaut des Auges 13 derart abbilden, dass die Abbildung des Diapositivs bzw. das virtuelle Target für den Probanden unter einer bestimmten virtuellen Entfernung erscheint. Die Stellung der zumindest einen sphärischen Linse zum Dia und bei mehreren Linsen auch zueinander erlaubt es, die virtuelle Position des virtuellen Targets bzw. die virtuelle Entfernung des virtuellen Targets von dem zumindest einen Auges 13 des Probanden und damit die Akkommodationsstimulanz des Auges 13 zu steuern.

So kann beispielsweise die virtuelle Position des virtuellen Targets derart eingestellt werden, dass eine Nahakkommodation (relativ geringe virtuelle Entfernung des virtuellen Targets) oder eine Fernakkommodation (relativ hohe virtuelle Entfernung des virtuellen Targets) stimuliert wird. Entsprechend ist es auch möglich, die virtuelle Entfernung des virtuellen Targets auf einen hinreichend kleinen oder hinreichend großen Wert einzustellen, so dass das Auge 13 auf die Abbildung nicht mehr akkommodieren kann, diese also nur als unscharf erkannt wird. Folglich stellt sich ein entspannter oder genebelter Zustand für das zumindest eine Auge 13 des Probanden ein. Das gezielte Hervorrufen eines solchen Zustands wird als "Nebelung" bezeichnet. Dazu ist es naturgemäß ausreichend, mit lediglich sphärischen Wirkungen zu arbeiten, da diese bereits ausreichen, um einem (fehlsichtigen) Auge eine Abbildung darzubieten, die in allen Richtungen nur unscharf wahr genommen werden kann, selbst wenn im Auge eine astigmatische Komponente der Fehlsichtigkeit vorliegt. Es muss lediglich eine Abbildung gewählt werden, die hinreichend jenseits des stärker positiven Hauptschnitts liegt.

Obwohl es möglich ist, Refraktionsmessungen auf Basis eines virtuellen Targets mittels Linsen, die eine lediglich sphärische Wirkung haben, durchzuführen, weist die Akkommodationsstimulanzeinrichtung 10 zusätzlich zu oder an Stelle der zumindest einen sphärischen Linse zumindest eine Zylinderlinse, vorzugsweise eine Vielzahl von Zylinderlinsen (z.B. ein Revolvermagazin von Zylinderlinsen) oder ein Linsensystem mit einstellbarer zylindrischer Wirkung auf, welche dem zumindest einen Auge 13 des Probanden vorgehalten werden kann/können.

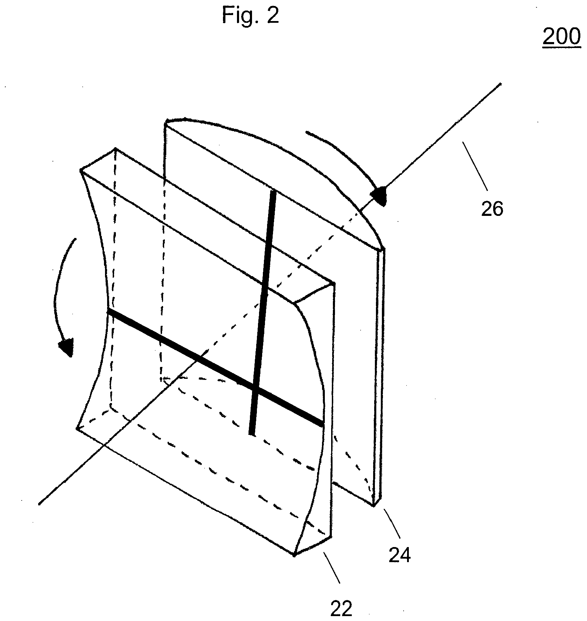

In **Fig. 2** ist ein solches Linsensystem mit einstellbarer zylindrischer Wirkung beispielhaft dargestellt. Gemäß der vorliegenden Erfindung wird also ein Target benutzt, das nicht nur eine sphärische Wirkung aufweist, sondern zusätzlich eine - nach Betrag und Richtung - anpassbare zylindrische Wirkung.

Insbesondere für Probanden mit einer hohen astigmatischen Komponente in der Fehlsichtigkeit erscheint ein klassisches Target nicht vollständig und in jeder Richtung gleichzeitig scharf. Je nach eingestellter optischer Wirkung des Targets und des angenommenen Akkommodationszustandes ist es entweder "im Mittel" scharf (bei Kompensation des sphärischen Äquivalentes) oder in beispielsweise der Richtung einer der beiden Hauptschnitte bei Kompensation der Wirkung in dem jeweiligen Hauptschnitt. Liegt die virtuelle Entfernung dabei in einem Bereich, der bei entsprechenden Akkommodationszuständen mit beiden Hauptschnitten scharf wahrgenommen werden kann, kann es auch zum Springen zwischen diesen beiden Akkommodationszuständen kommen, d.h. das Auge kann während der Messung oder in aufeinander folgenden Messungen bei der selben virtuellen Entfernung unterschiedliche Akkommodationszustände einnehmen, was zu unterschiedlichen und damit ungenauen Werten der zu (objektiv) zu bestimmenden ophthalmologischen Daten führt.

Durch die erfindungsgemäße Verwendung eines optischen Systems (z.B. von Linsen) mit zylindrischer Wirkung eines virtuellen Targets kann eine astigmatische Fehlsichtigkeit des zumindest einen Auges des Probanden zumindest teilweise kompensiert werden. Dem Proband wird eine Abbildung bzw. ein virtuelles Target vorgesetzt, die/das er vollständig (abgesehen von der in diesem Zusammenhang vernachlässigbaren Aberrationen höherer Ordnung) scharf wahrnehmen kann. Dadurch wird ihm im Vergleich zu einem nicht kompensierten Astigmatismus des zumindest einen Auges 13 des Probanden sowohl die Fixation als auch die Akkommodation erleichtert. Refraktionsmessungen auf Basis eines virtuellen Targets können folglich genauer und verlässlicher durchgeführt werden, da es bei solchen Messungen entscheidend darauf ankommt, dass der Proband auch tatsächlich auf das Target akkommodiert. Insbesondere bei Probanden mit einer hohen astigmatischen Fehlsichtigkeit ist dies ohne eine entsprechende Kompensation nicht immer gewährleistet, was zu Fehlern in der Refraktionsbestimmung führen kann.

Außerdem können durch die Erfindung in vielen Fällen Ermüdungserscheinungen des Auges, die bei herkömmlichen (objektiven) Refraktionsbestimmungen aufgrund eines möglicherweise unbefriedigenden Akkommodationserfolgs auftreten können, deutlich reduziert werden. Dadurch begünstigt die vorliegende Erfindung auch eine schnellere Akkommodation des Auges, was die schnellere Durchführung einzelner Messungen und damit - falls gewünscht - die Durchführung von mehr Messungen, eventuell bei unterschiedlichen virtuellen Entfernungen (Akkommodationszuständen) vorzugsweise sogar ohne eine Verlängerung der Gesamtmesszeit ermöglicht. Auch dadurch kann wiederum die Messgenauigkeit erhöht werden.

Die bevorzugte Vorrichtung zur Bestimmung ophthalmologischer Daten, wie sie in Fig. 1 gezeigt ist, weist ferner eine Messeinrichtung auf, mit der die ophthalmologischen Daten gemessen bzw. erfasst werden können. Die Messeinrichtung kann vorzugsweise derjenigen eines herkömmlichen Autorefraktometers oder Aberrometers entsprechen. Die Messeinrichtung umfasst gemäß der in Fig. 1 dargestellten bevorzugten Ausführungsform im Wesentlichen einen Laser 16, einen zweiten Strahlteiler 14 (teildurchlässiger Spiegel), sowie einen Detektor 18. Mit Hilfe eines räumlich begrenzten, insbesondere fokussierten Laserstrahls, der vom Laser 16 emittiert und durch den zweiten Strahlteiler 14 in das Auge 13 gelenkt wird, wird im Auge 13 von dem beleuchteten Punkt auf der Netzhaut ausgehend eine Kugelwelle induziert, die im Auge (insbesondere an den Grenzflächen) und an dessen Oberfläche gebrochen wird. Je nach den optischen Eigenschaften des Auges verlässt das Licht das Auge mit Wellenfronten, die von einer Kugelform mehr oder weniger abweichen können. Diese Wellenfronten breiten sich durch die beiden Strahlteiler 12 und 14 hindurch aus und werden schließlich vom Detektor 18 detektiert. Aus dem detektierten Signal der vom Auge ausgesandten Wellenfront können zur Refraktionsbestimmung ophthalmologische Daten wie beispielsweise Sphäre, Astigmatismus, und insbesondere im Falle eines Aberrometers auch Abbildungsfehler höherer Ordnung des untersuchten Auges ermittelt bzw. erfasst werden. Ferner können auch weitere ophthalmologische Daten, wie z.B. die Pupillengröße oder bei Verwenden eines Shack-Hartmann Sensors die Wellenfront, gemessen werden.

Fig. 2 zeigt eine schematische Darstellung eines Linsensystems, welches neben einer sphärischen Wirkung auch eine einstellbare astigmatische Wirkung aufweist. Das Linsensystem (auch bekannt unter Stokessche Linse) besteht aus zwei Planzylinder mit entgegengesetzt gleicher optischer Wirkung (negativer Planzylinder 22 und positiver Planzylinder 24), die in einer Fassung (hier nicht gezeigt) gegeneinander um eine gemeinsame optische Achse 26 drehbar montiert sind. Liegen die beiden Zylinderachsen (dargestellt durch dicke schwarze Linien in Fig. 2) parallel zueinander, so kompensieren sich die Wirkungen der zwei Zylinder, und beide zusammen wirken zumindest näherungsweise wie eine planparallele Platte. Stehen die Achsen jedoch senkrecht zueinander (wie in Fig. 2 skizziert), so entsteht eine sphäro-zylindrische Kombination. Dabei ergibt sich die sphärische Wirkung aus der Wirkung des Einzelzylinders. Die sogenannte "astigmatische Differenz" ergibt sich aus der Summe der Beträge der inversen Krümmungsradien der Einzelzylinder. Durch kontinuierliches Verdrehen der Zylinderachsen lassen sich unterschiedliche Zwischenwerte der astigmatischen Differenz einstellen.

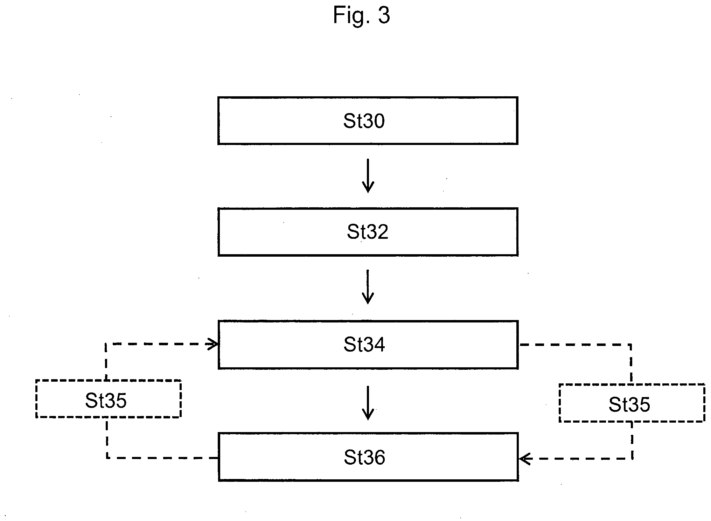

In Fig. 3 ist ein Ablauf eines Verfahrens zur Bestimmung ophthalmologischer Daten, insbesondere zur Refraktionsbestimmung, gemäß einer bevorzugten Ausführungsform der vorliegenden Erfindung schematisch dargestellt. Vorzugsweise umfasst das Verfahren zumindest zwei Messungen, eine erste Messung (Vormessung) und zumindest eine zweite Messung (Hauptmessung). Unter einer Messung wird hierbei allgemein das Erfassen ophthalmologischer Daten verstanden.

Um eine erste Messung, d.h. eine Vormessung durchzuführen, wird zunächst in einem ersten Schritt St30 ein virtuelles Target mit sphärischer und einstellbarer astigmatischer Wirkung in das zumindest eine Auge des Probanden projiziert. Da zu diesem Zeitpunkt möglicherweise noch keine Informationen zur Fehlsichtigkeit des Probanden vorliegen, wird vorzugsweise das virtuelle Target auf eine virtuelle Position eingestellt, welche einen genebelten Zustand bewirkt. Die zylindrische Wirkung des virtuellen Targets wird vorzugsweise neutral, d.h. auf Null eingestellt.

In einem nächsten Schritt St32 der beschriebenen bevorzugten Ausführungsform wird zur groben Bestimmung der Fehlsichtigkeit, insbesondere der astigmatischen Fehlsichtigkeit, des zumindest einen Auges 13 des Probanden eine Vormessung durchgeführt. Dieser Schritt St32 beinhaltet also vorzugsweise das Erfassen eines ersten Wertes einer astigmatischen Fehlsichtigkeit des zumindest einen Auges des Probanden in einer ersten Messung (Vormessung). In dieser Vormessung können vorzugsweise auch weitere ophthalmologische Daten, wie beispielsweise die Sphäre, gemessen werden. Besonders bevorzugt wird in der Vormessung der gleiche Satz ophthalmologischer Daten wie in der Hauptmessung erfasst.

In einem nachfolgenden Schritt St34 der beschriebenen bevorzugten Ausführungsform wird die zylindrische Wirkung des virtuellen Targets auf einen Wert eingestellt, der den erfassten ersten Wert der astigmatischen Fehlsichtigkeit des zumindest einen Auges des Probanden im Wesentlichen kompensiert. Dadurch wird erreicht, dass der Proband das virtuelle Target im Wesentlichen ohne astigmatische Fehler und damit in allen Richtungen im Wesentlichen gleichmäßig scharf wahrnehmen kann.

In der darauf folgenden Hauptmessung St36, welche vorzugsweise zunächst ebenfalls im genebelten Zustand durchgeführt werden kann, wird somit erreicht, dass der Proband das virtuelle Target in allen Richtungen gleichmäßig unscharf wahrnimmt und keine Strukturen erkennt. Dies ist für den Probanden wesentlich angenehmer und gewährleistet zugleich eine gute Stabilität der Akkommodation im genebelten Zustand. Mit der Hauptmessung werden insbesondere Sphäre und/oder Astigmatismus des zumindest einen Auges des Probanden erfasst. Es können mit der Hauptmessung aber auch zusätzlich beliebige andere ophthalmologische Daten erfasst werden. Hat sich der aus der Hauptmessung ermittelte Wert für den Astigmatismus im Vergleich zur Vormessung geändert, so können zur weiteren Verbesserung der Refraktionsbestimmung unmittelbar die Schritte St34 und St36 wiederholt werden.

Nach dem Schritt St34 kann vor dem Durchführen der Hauptmessung in einem optionalen (in Figur 3 gestrichelt dargestellt) Schritt St35 die Position (d.h. die virtuelle Entfernung) des virtuellen Targets geändert werden. Somit ist es möglich, die Hauptmessung nicht im genebelten Zustand, sondern bei einer bestimmten durch das virtuelle Target stimulierten Akkommodation des zu messenden Auges 13 durchzuführen. Beispielsweise kann eine Messung im fernakkommodierten Zustand (Fernmessung) oder eine Messung im nahakkommodierten Zustand (Nahmessung) durchgeführt werden. Durch das im Schritt St34 vorgenommene Einstellen der astigmatischen Wirkung auf Basis der Vormessung wird mit hoher Wahrscheinlichkeit gewährleistet, dass der Proband während der Hauptmessung auch tatsächlich das virtuelle Target fixiert bzw. darauf akkommodiert. Dies macht die Messung zuverlässiger und genauer.

Nachdem die Hauptmessung mit Schritt St36 durchgeführt wurde, kann anschließend die Position des virtuellen Targets geändert werden (Schritt St35), um zumindest eine weitere Hauptmessung für einen anderen Akkommodationszustand des Auges 13 durchzuführen. Vorzugsweise wird zwischen den einzelnen Hauptmessungen die virtuelle Entfernung des virtuellen Targets sukzessive (d.h. monoton) verringert. Vorzugsweise wird vor jeder Hauptmessung durch Schritt St35 die einstellbare zylindrische Wirkung des virtuellen Targets auf einen Wert eingestellt, der den bei der vorangegangenen Messung erfassten Wert der astigmatischen Fehlsichtigkeit des zumindest einen Auges des Probanden im Wesentlichen kompensiert. Damit wird erreicht, dass für jede Hauptmessung die astigmatische Fehlsichtigkeit des Probanden im Wesentlichen kompensiert wird und somit das Ergebnis der Refraktionsbestimmung verbessert wird.

**Bezugszeichenliste**

| | |
|---|---|
| 10 | Akkommodationsstimulanzeinrichtung |
| 12 | erster Strahlteiler |
| 13 | Auge |
| 14 | zweiter Strahlteiler |
| 16 | Laser / Laserdiode |
| 18 | Detektor |
| 22 | negativer Planzylinder |
| 24 | positiver Planzylinder |
| 26 | optische Achse |
| 100 | Vorrichtung zur Bestimmung ophthalmologischer Daten |
| 200 | Linsensystem/Stokessche Linse als Astigmatismuskompensator |
| St30 | Projizieren eines virtuellen Targets mit sphärischer und einstellbarer astigmatischer Wirkung in das zumindest eine Auge des Probanden |
| St32 | Vormessung (erste Messung) |
| St34 | Einstellen der astigmatischen Wirkung des virtuellen Targets |
| St35 | Ändern der virtuellen Position des virtuellen Targets (optional) |
| St36 | Hauptmessung (zweite, dritte, vierte, usw., Messung) |

**Patentansprüche**

1. Vorrichtung (100) zur Bestimmung eines Satzes ophthalmologischer Daten von zumindest einem Auge (13) eines Probanden, aufweisend:

   - eine Akkommodationsstimulanzeinrichtung (10), welche ausgelegt ist, ein virtuelles Target mit einer sphärischen Wirkung und mit einer einstellbaren zylindrischen Wirkung in das zumindest eine Auge (13) des Probanden zu projizieren; und
   - eine Messeinrichtung (14, 16, 18) zum Erfassen ophthalmologischer Daten des zumindest einen Auges (13) des Probanden, wobei

   die Messeinrichtung ausgelegt ist, einen Wert einer astigmatischen Refraktion des zumindest einen Auges (13) des Probanden zu erfassen und wobei die Vorrichtung eine Steuereinrichtung umfasst, welche ausgelegt ist, die sphärische Wirkung des virtuellen Targets von einer ersten sphärischen Wirkung zu einer zweiten sphärischen Wirkung zu verändern, während die Akkommodationsstimulanzeinrichtung (10) das virtuelle Target mit einer zylindrischen Wirkung in das zumindest eine Auge (13) des Probanden derart projiziert, dass die zylindrische Wirkung des virtuellen Targets den erfassten Wert der astigmatischen Fehlsichtigkeit des zumindest einen Auges (13) zumindest teilweise kompensiert.

2. Vorrichtung (100) nach Anspruch 1, wobei die Akkommodationsstimulanzeinrichtung (10) zumindest eine Linse mit einer sphärischen Wirkung und zumindest eine Linse mit einer zylindrischen Wirkung aufweist.

3. Vorrichtung (100) nach Anspruch 1 oder 2, wobei die Akkommodationsstimulanzeinrichtung (10) ein Magazin mit einer Vielzahl von Zylinderlinsen umfasst, welche jeweils unterschiedliche zylindrische Wirkungen aufweisen, und wobei das Magazin derart ausgelegt und angeordnet ist, dass einzelne Zylinderlinsen und/oder eine Kombination mehrerer Zylinderlinsen des Magazins auswählbar und zum Projizieren des virtuellen Targets verwendbar sind.

4. Vorrichtung (100) nach einem der vorangegangenen Ansprüche, wobei die Akkommodationsstimulanzeinrichtung (10) ein Alvarez-Linsensystem aufweist.

5. Vorrichtung (100) nach einem der vorangegangenen Ansprüche, wobei die Akkommodationsstimulanzeinrichtung (10) zwei gegeneinander verdrehbare Linsen mit jeweils zumindest einer zylindrischen Komponente in den Wirkungen umfasst.

6. Vorrichtung (100) nach Anspruch 5, wobei die Akkommodationsstimulanzeinrichtung (10) zwei Zylinderlinsen mit ineinander fassenden, einander zugewandten rotationssymmetrischen Flächen, vorzugsweise Planflächen, aufweist.

7. Vorrichtung (100) nach Anspruch 5 oder 6, wobei die Akkommodationsstimulanzeinrichtung eine positive (24) und eine negative (22) Zylinderlinse mit entgegengesetzt gleicher Wirkung aufweist, welche gegeneinander drehbar gelagert und vorzugsweise gegeneinander verschiebbar sind.

8. Vorrichtung (100) nach einem der vorangegangenen Ansprüche, wobei die Vorrichtung als Aberrometer und/oder als Autorefraktometer ausgestaltet ist.

9. Verfahren zur Bestimmung eines Satzes ophthalmologischer Daten von zumindest einem Auge (13) eines Probanden, umfassend folgende Schritte:

   - Erfassen eines ersten Wertes einer astigmatischen Refraktion des zumindest einen Auges (13) des Probanden;
   - Projizieren eines virtuellen Targets mit einer ersten sphärischen Wirkung und mit einer ersten zylindrischen Wirkung in das zumindest eine Auge (13) des Probanden derart, dass die erste zylindrische Wirkung des virtuellen Targets den erfassten ersten Wert der astigmatischen Fehlsichtigkeit des zumindest einen Auges zumindest teilweise kompensiert;
   - Verändern der sphärischen Wirkung des virtuellen Targets von der ersten sphärischen Wirkung zu einer zweiten sphärischen Wirkung, während das virtuelle Target mit einer zylindrischen Wirkung in das zumindest eine Auge (13) des Probanden derart projiziert wird, dass die zylindrische Wirkung des virtuellen Targets den erfassten ersten Wert der astigmatischen Fehlsichtigkeit des zumindest einen Auges (13) zumindest teilweise kompensiert; und
   - Erfassen der ophthalmologischen Daten des zumindest einen Auges, während das Auge zur Akkommodation auf das virtuelle Target stimuliert wird.

10. Verfahren nach Anspruch 9, wobei das Erfassen des ersten Wertes einer astigmatischen Fehlsichtigkeit des zumindest einen Auges (13) ein Messen der astigmatischen Fehlsichtigkeit des Auges des Probanden insbesondere bei einer Fernakkommodation des Auges und/oder im genebelten Zustand umfasst.

11. Verfahren nach Anspruch 9 oder 10, wobei das Erfassen der ophthalmologischen Daten des zumindest einen Auges (13) des Probanden bei einer Nahakkommodation des zumindest einen Auge (13) des Probanden erfolgt.

12. Verfahren nach einem der Ansprüche 9 bis 11, wobei das Erfassen der ophthalmologischen Daten ein Erfassen eines zweiten Wertes der astigmatischen Fehlsichtigkeit des zumindest einen Auges des Probanden umfasst und wobei das Verfahren ferner umfasst:

   - Projizieren des virtuellen Targets mit einer zweiten sphärischen Wirkung und einer zweiten zylindrischen Wirkung in das zumindest eine Auge (13) des Probanden derart, dass die zweite zylindrische Wirkung des virtuellen Targets den erfassten zweiten Wert der astigmatischen Fehlsichtigkeit des zumindest einen Auges zumindest teilweise kompensiert; und
- Erfassen weiterer ophthalmologischer Daten des zumindest einen Auges (13) des Probanden.

13. Verfahren nach einem der Ansprüche 9 bis 12, wobei das virtuelle Target bei der ersten sphärischen Wirkung eine größere virtuelle Objektentfernung simuliert als bei der zweiten sphärischen Wirkung.

## Claims

1. Device (100) for determining a set of ophthalmological data of at least one eye (13) of a subject, comprising:

   - an accommodation stimulation apparatus (10) configured to project a virtual target with a spherical power and with an adjustable cylindrical power into the at least one eye (13) of the subject; and
   - a measuring apparatus (14, 16, 18) for registering ophthalmological data of the at least one eye (13) of the subject, wherein

   the measuring apparatus is configured to register a value of an astigmatic refraction of the at least one eye (13) of the subject and wherein the device comprises a control apparatus configured to change the spherical power of the virtual target from a first spherical power to a second spherical power while the accommodation stimulation apparatus (10) projects the virtual target with a cylindrical power into the at least one eye (13) of the subject in such a way that the cylindrical power of the virtual target at least partly compensates the registered value of the astigmatic refractive error of the at least one eye (13).

2. Device (100) according to Claim 1, wherein the accommodation stimulation apparatus (10) has at least one lens with a spherical power and at least one lens with a cylindrical power.

3. Device (100) according to Claim 1 or 2, wherein the accommodation stimulation apparatus (10) comprises a magazine with a multiplicity of cylindrical lenses, which each have different cylindrical powers, and wherein the magazine is configured and arranged in such a way that individual cylindrical lenses and/or a combination of a plurality of cylindrical lenses of the magazine are selectable and employable for projecting the virtual target.

4. Device (100) according to one of the preceding claims, wherein the accommodation stimulation apparatus (10) has an Alvarez lens system.

5. Device (100) according to one of the preceding claims, wherein the accommodation stimulation apparatus (10) comprises two lenses which are rotatable in relation to one another and which respectively have at least one cylindrical component in the powers.

6. Device (100) according to Claim 5, wherein the accommodation stimulation apparatus (10) has two cylindrical lenses with mutually engaging, mutually facing rotationally symmetric surfaces, preferably plane surfaces.

7. Device (100) according to Claim 5 or 6, wherein the accommodation stimulation apparatus has a positive (24) and a negative (22) cylindrical lens with equal and opposite powers, said cylindrical lenses being rotatably mounted in relation to one another and preferably being displaceable in relation to one another.

8. Device (100) according to one of the preceding claims, wherein the device is embodied as an aberrometer and/or as an automated refractor.

9. Method for determining a set of ophthalmological data of at least one eye (13) of a subject, comprising the following steps:

   - registering a first value of an astigmatic refraction of the at least one eye (13) of the subject;
   - projecting a virtual target with a first spherical power and with a first cylindrical power into the at least one eye (13) of the subject in such a way that the first cylindrical power of the virtual target at least partly compensates the registered first value of the astigmatic refractive error of the at least one eye;
   - changing the spherical power of the virtual target from the first spherical power to a second spherical power while the virtual target with a cylindrical power is projected into the at least one eye (13) of the subject in such a way that the cylindrical power of the virtual target at least partly compensates the registered first value of the astigmatic refractive error of the at least one eye (13); and
- registering the ophthalmological data of the at least one eye while the eye is stimulated to accommodate to the virtual target.

10. Method according to Claim 9, wherein the registration of the first value of an astigmatic refractive error of the at least one eye (13) comprises a measurement of the astigmatic refractive error of the eye of the subject, particularly during a far accommodation of the eye and/or in the hazy state.

11. Method according to Claim 9 or 10, wherein the registration of the ophthalmological data of the at least one eye (13) of the subject is implemented during a near accommodation of the at least one eye (13) of the subject.

12. Method according to one of Claims 9 to 11, wherein the registration of the ophthalmological data comprises a registration of a second value of the astigmatic refractive error of the at least one eye of the subject and wherein the method furthermore comprises:

- projecting the virtual target with a second spherical power and a second cylindrical power into the at least one eye (13) of the subject in such a way that the second cylindrical power of the virtual target at least partly compensates the registered second value of the astigmatic refractive error of the at least one eye; and
- registering further ophthalmological data of the at least one eye (13) of the subject.

13. Method according to one of Claims 9 to 12, wherein the virtual target simulates a greater virtual object distance in the case of the first spherical power than in the case of the second spherical power.


**Revendications**

1. Dispositif (100) de détermination d'un ensemble de données ophtalmologiques d'au moins un oeil (13) d'un patient examiné, comprenant :

- un système de stimulation de l'accommodation (10), lequel est conçu pour projeter une cible virtuelle ayant un effet sphérique et ayant un effet cylindrique réglable dans ledit au moins un oeil (13) du patient examiné ; et
- un système de mesure (14, 16, 18) destiné à acquérir les données ophtalmologiques dudit au moins un oeil (13) du patient examiné, dans lequel

le système de mesure est conçu pour acquérir une valeur d'une réfraction astigmatique dudit au moins un oeil (13) du patient examiné et dans lequel le dispositif comporte un système de commande, lequel est conçu pour modifier l'effet sphérique de la cible virtuelle d'un premier effet sphérique à un deuxième effet sphérique, pendant que le système de stimulation de l'accommodation (10) projette la cible virtuelle ayant un effet cylindrique dans ledit au moins un oeil (13) du patient examiné, de telle sorte que l'effet cylindrique de la cible virtuelle compense au moins en partie la valeur acquise de l'amétropie astigmatique dudit au moins un oeil (13).

2. Dispositif (100) selon la revendication 1, dans lequel le système de stimulation de l'accommodation (10) comprend au moins une lentille ayant un effet sphérique et au moins une lentille ayant un effet cylindrique.

3. Dispositif (100) selon la revendication 1 ou 2, dans lequel le système de stimulation de l'accommodation (10) comporte un magasin pourvu d'une pluralité de lentilles cylindriques, lesquelles présentent chacune différents effets cylindriques, et dans lequel le magasin est conçu et agencé de telle manière que différentes lentilles cylindriques et/ou une combinaison de plusieurs lentilles cylindriques du magasin peuvent être sélectionnées et utilisées pour projeter la cible virtuelle.

4. Dispositif (100) selon l'une quelconque des revendications précédentes, dans lequel le système de stimulation de l'accommodation (10) comprend un système de lentilles Alvarez.

5. Dispositif (100) selon l'une quelconque des revendications précédentes, dans lequel le système de stimulation de l'accommodation (10) comporte deux lentilles pouvant tourner l'une par rapport à l'autre pourvues chacune d'au moins une composante cylindrique dans les effets.

**6.** Dispositif (100) selon la revendication 5, dans lequel le système de stimulation de l'accommodation (10) comprend deux lentilles cylindriques pourvues de surfaces à symétrie de rotation s'imbriquant l'une dans l'autre, tournées l'une vers l'autre, de préférence des surfaces planes.

**7.** Dispositif (100) selon la revendication 5 ou 6, dans lequel le système de stimulation de l'accommodation (10) comprend une lentille cylindrique positive (24) et une lentille cylindrique négative (22) présentant un effet identique mais de sens opposé, lesquelles sont montées de manière à pouvoir tourner l'une par rapport à l'autre et lesquelles peuvent coulisser de préférence l'une par rapport à l'autre.

**8.** Dispositif (100) selon l'une quelconque des revendications précédentes, dans lequel le dispositif est réalisé sous la forme d'un aberromètre et/ou d'un autoréfractomètre.

**9.** Procédé de détermination d'un ensemble de données ophtalmologiques d'au moins un oeil (13) d'un patient examiné, comprenant les étapes suivantes :

   - l'acquisition d'une première valeur d'une réfraction astigmatique dudit au moins un oeil (13) du patient examiné ;
   - la projection d'une cible virtuelle ayant un premier effet sphérique et un premier effet cylindrique dans ledit au moins un oeil (13) du patient examiné, de telle sorte que le premier effet cylindrique de la cible virtuelle compense au moins en partie la première valeur acquise de l'amétropie astigmatique dudit au moins un oeil ;
   - la modification de l'effet sphérique de la cible virtuelle du premier effet sphérique au deuxième effet sphérique, pendant que la cible virtuelle ayant un effet cylindrique est projetée dans ledit au moins un oeil (13) du patient examiné, de telle sorte que l'effet cylindrique de la cible virtuelle compense au moins en partie la première valeur acquise de l'amétropie astigmatique dudit au moins un oeil (13) ; et
   - l'acquisition des données ophtalmologiques dudit au moins un oeil, pendant que l'oeil est stimulé sur la cible virtuelle pour l'accommodation.

**10.** Procédé selon la revendication 9, dans lequel l'acquisition de la première valeur d'une amétropie astigmatique dudit au moins un oeil (13) comprend la mesure de l'amétropie astigmatique de l'oeil du patient examiné en particulier pour une accommodation de l'oeil en vision de loin et/ou à l'état dilaté.

**11.** Procédé selon la revendication 9 ou 10, dans lequel l'acquisition des données ophtalmologiques dudit au moins un oeil (13) du patient examiné est réalisée lors d'une accommodation en vision de près dudit au moins un oeil (13) du patient examiné.

**12.** Procédé selon l'une quelconque des revendications 9 à 11, dans lequel l'acquisition des données ophtalmologiques comprend l'acquisition d'une deuxième valeur de l'amétropie astigmatique dudit au moins un oeil du patient examiné et le procédé comprenant en outre :

   - la projection de la cible virtuelle ayant un deuxième effet sphérique et un deuxième effet cylindrique dans ledit au moins un oeil (13) du patient examiné, de telle sorte que le deuxième effet cylindrique de la cible virtuelle compense au moins en partie la deuxième valeur acquise de l'amétropie astigmatique dudit au moins un oeil ; et
   - l'acquisition d'autres données ophtalmologiques dudit au moins un oeil (13) du patient examiné.

**13.** Procédé selon l'une quelconque des revendications 9 à 12, dans lequel la cible virtuelle simule une plus grande distance virtuelle par rapport à un objet dans le cas du premier effet sphérique que dans le cas du deuxième effet sphérique.

Fig. 1

Fig. 2

200

22

24

26

EP 2 943 114 B1

Fig. 3

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 2012287398 A1

- WO 2010065475 A2

**In der Beschreibung aufgeführte Nicht-Patentliteratur**

- **ALLERGAN HUMPHREY.** Das Humphrey-Refraktometer. *Produktinformationen,* Juli 2012, http://www.ophthalworld.de/cosmoshop/pix/a/media/21062012/Prospekt%20Autoref%20580_%20585_%20590.pdf
- **K. NICKE ; S. TRUMM.** Brillengläser der Zukunft - Schritt 3 Der DNEye Scanner. *Der Augenoptiker,* Juni 2012

- **H. PAUL.** Lexikon der Optik. Spektrum Akademischer Verlag GmbH, 2003
- **BREITENSTEIN.** Allgemeine Optik. *Skript zur Vorlesung an der HFA Köln,* 2004, www.hfak.de/dozenten/breitenstein/Skripte/AlOp1.pdf